# EUROPEAN PATENT SPECIFICATION

(11) **EP 3 986 871 B1**
(45) Date of publication and mention of the grant of the patent: **07.06.2023**
(21) Application number: 20733429.3
(22) Date of filing: 18.06.2020
(51) Int. Cl.: C07D 239/95, A61K 31/517, A61P 31/00, A61P 37/00

(54) **QUINAZOLINE PRODRUGS FOR THE TREATMENT OF VIRAL INFECTIONS AND FURTHER DISEASES**
CHINAZOLIN-PRODRUGS ZUR BEHANDLUNG VON VIRUSINFEKTIONEN UND WEITEREN ERKRANKUNGEN
PROMÉDICAMENTS À BASE DE QUINAZOLINE POUR LE TRAITEMENT D'INFECTIONS VIRALES ET D'AUTRES MALADIES

(30) Priority: 18.06.2019 EP 19180951
(43) Date of publication of application: 27.04.2022
(73) Proprietor: Janssen Sciences Ireland Unlimited Company, Ringaskiddy, Co Cork (IE)
(72) Inventor: MC GOWAN, David Craig, 2340 Beerse (BE)
(74) Representative: Paris, Fabienne
(86) International application number: PCT/EP2020/066896
(87) International publication number: WO 2020/254473

(56) References cited:
- WO-A1-2012/156498
- WO-A1-2016/141092
- WO-A1-2017/163264
- PAUL BARNALI ET AL: "Activity-guided development of potent and selective toll-like receptor 9 antagonists", EUROPEAN JOURNAL OF MEDICINAL CHEMISTRY, ELSEVIER, AMSTERDAM, NL, vol. 159, 26 September 2018 (2018-09-26), pages 187-205, XP085505177, ISSN: 0223-5234, DOI: 10.1016/J.EJMECH.2018.09.058
- WERNER EMBRECHTS ET AL: "2,4-Diaminoquinazolines as Dual Toll-like Receptor (TLR) 7/8 Modulators for the Treatment of Hepatitis B Virus", JOURNAL OF MEDICINAL CHEMISTRY, vol. 61, no. 14, 2 July 2018 (2018-07-02), pages 6236-6246, XP055590264, US ISSN: 0022-2623, DOI: 10.1021/acs.jmedchem.8b00643

## Description

### BACKGROUND

The treatment of viral infections, immune disorders, and cancers can be facilitated through induction of the T helper 1 (Th1) immune response. Vaccine adjuvants also utilize the Th1 response to increase the effectiveness of the vaccine. Therefore, compounds that promote Th1 responses are desired therapeutics.

For instance, in the treatment of chronic hepatitis B (HBV), a Th1 response to reinvigorate exhausted virus-specific CD8⁺ T cells in the infected organs would be highly beneficial *(*Science 1999, 284, 825-829; J. Virol. 2003, 77, 68-76). Such a response is induced by the innate immune system, in particular by stimulating Toll-like receptors (TLR) such as TLR3, 4, 7, 8 and 9 *(*Clin. Transl. Immunol. 2016, 5(5):e85). TLR8 agonists induce one of the strongest Th1 responses in human cells, through the secretion of IL-12p70, the upregulation of CD40 or OX40L activation markers, and indirectly through the upregulation of IFNγ (J. Immunol. 2006, 176(12): 7438-46; Hum. Immunol. 2011 Jan; 72(1): 24-31; J. Leukoc. Biol. 2012, 91(1): 105-17). These factors have shown potential to treat chronic HBV *ex vivo (*PLoS Pathog. 2013, 9, e1003208; J. Exp. Med. 2014, 211, 2047-2059; PLoS Path. 2013, 9, e1003490; PLoS Pathog. 2014, 10, e1004210).

Only one TLR8 agonist, administered by subcutaneous injection, is currently in development for cancer indications *(*Clin. Cancer Res. 2014, 20, 3683; Cancer Immunol. Immunother. 2013, 62, 1347; WO 2012/045089. See also US 2008/234251, US 2010/029585). Therefore, there exists a strong need for orally-available TLR8 agonists to treat infections such as chronic HBV.

### SUMMARY

The present disclosure is directed toward quinazoline prodrugs and methods of treating infections and diseases with quinazoline prodrugs.

In an aspect, provided herein is a compound of Formula I: 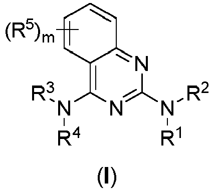 or a pharmaceutically acceptable salt thereof;
wherein
R¹ is selected from the group consisting of hydrogen, C₁-C₃ alkyl, -C(O)C₁-C₃ alkyl, -C(O)OC₁-C₃ alkyl, and -P(O)(OC₁-C₃ alkyl)₂;
R² is selected from the group consisting of C₁-C₃ alkyl, -C(O)C₁-C₃ alkyl, -C(O)OC₁-C₃ alkyl, -P(O)(OC₁-C₃ alkyl)₂, -(CH₂)₁₋₃C(O)C₁-C₃ alkyl, -(CH₂)₁₋₃C(O)OC₁-C₃ alkyl, -(CH₂)₁₋₃P(O)(OC₁-C₃ alkyl)₂ and C₃₋₆heterocycle comprising one or more heteroatom(s), the one or more heteroatom(s) being selected from oxygen, nitrogen and sulfur, more particularly oxygen;
R³ is C₁-C₈ alkyl, wherein C₁-C₈ alkyl is optionally substituted by one or more substituents
selected from halogen, -OH, -NH₂, amino, nitrile, ester, amide, C₁₋₃ alkyl and C₁₋₃ alkoxy;
the carbon of R³ bonded to the amine in the 4-position of the quinazoline is in (R)-configuration,
R⁴ is selected from the group consisting of hydrogen, C₁-C₈ alkyl, -C(O)C₁-C₃ alkyl, and -C(O)OC₁-C₃ alkyl;
R⁵ is independently, at each occurrence, selected from the group consisting of hydrogen, C₁-C₃ alkyl, -OC₁-C₃ alkyl, and halogen;
with the proviso that not all 4 occurrences of R⁵ are hydrogen; and
m is 4.

In another aspect, provided herein are pharmaceutical compositions comprising a compound disclosed herein, or a pharmaceutically acceptable salt thereof, and one of more pharmaceutically acceptable carriers or excipients.

In yet another aspect, provided herein are compounds and pharmaceutical compositions for use in the treatment or prevention of a viral infection in a subject in need thereof.

In an embodiment, the viral infection is a hepatitis B (HBV) infection. In another embodiment, the compound or pharmaceutical composition disclosed herein is administered as a vaccine adjuvant.

In still another aspect, provided herein are compounds and pharmaceutical compositions for use in the treatment of an immune disorder in a subject in need thereof.

In an aspect, provided herein are compounds and pharmaceutical compositions for use in the treatment of cancer in a subject in need thereof.

### DETAILED DESCRIPTION

Provided herein are quinazoline prodrugs, or pharmaceutically acceptable salts thereof, that can be used for the treatment of infections and diseases such as a hepatitis B infection, immune disorders, and cancer. The compounds disclosed herein can also be utilized as vaccine adjuvants to increase the effectiveness of vaccines.

The present disclosure includes a prodrug composition of a pharmaceutical species. The pharmaceutical species is characterized by bioavailability of 50% or less and a molecular weight in the range of 100-1000 Daltons. Also described is a method of delivering a pharmaceutical species to an individual including the step of orally administering an inventive prodrug to an individual. The prodrug moiety is attached to the pharmaceutical species wherein the modification allows the TLR8 agonist potential to be attenuated. The prodrug is enzymatically cleaved prior to or at the site of the liver to yield the pharmaceutical species, such that the pharmaceutical species is delivered to the individual limiting TLR8 agonism prior to the liver.

### Definitions

Listed below are definitions of various terms used to describe the compounds provided herein. These definitions apply to the terms as they are used throughout this specification and claims, unless otherwise limited in specific instances, either individually or as part of a larger group.

Unless defined otherwise, all technical and scientific terms used herein generally have the same meaning as commonly understood by one of ordinary skill in the art. Generally, the nomenclature used herein and the laboratory procedures in cell culture, molecular genetics, organic chemistry, and peptide chemistry are those well-known and commonly employed in the art.

As used herein, the articles "a" and "an" refer to one or to more than one (i.e., to at least one) of the grammatical object of the article. By way of example, "an element" means one element or more than one element. Furthermore, use of the term "including" as well as other forms, such as "include," "includes," and "included," is not limiting.

As used herein, the term "about" will be understood by persons of ordinary skill in the art and will vary to some extent on the context in which it is used. As used herein when referring to a measurable value such as an amount, a temporal duration, and the like, the term "about" is meant to encompass variations of ±20% or ±10%, including ±5%, ±1%, and ±0.1% from the specified value, as such variations are appropriate to perform the disclosed methods.

The term "treat," "treated," "treating," or "treatment" includes the diminishment or alleviation of at least one symptom associated or caused by the state, disorder or disease being treated.

As used herein, the term "prevent" or "prevention" means no disorder or disease development if none had occurred, or no further disorder or disease development if there had already been development of the disorder or disease. Also considered is the ability of one to prevent some or all of the symptoms associated with the disorder or disease.

As used herein, the term "patient," "individual," or "subject" refers to a human or a non-human mammal. Non-human mammals include, for example, livestock and pets, such as ovine, bovine, porcine, canine, feline and marine mammals. Preferably, the patient, subject, or individual is human.

As used herein, the terms "effective amount," "pharmaceutically effective amount," and "therapeutically effective amount" refer to a nontoxic but sufficient amount of an agent to provide the desired biological result. That result may be reduction or alleviation of the signs, symptoms, or causes of a disease, or any other desired alteration of a biological system. An appropriate therapeutic amount in any individual case may be determined by one of ordinary skill in the art using routine experimentation.

As used herein, the term "pharmaceutically acceptable" refers to a material, such as a carrier or diluent, which does not abrogate the biological activity or properties of the compound, and is relatively non-toxic, i.e., the material may be administered to an individual without causing undesirable biological effects or interacting in a deleterious manner with any of the components of the composition in which it is contained.

As used herein, the term "pharmaceutically acceptable salt" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the compounds provided herein include the conventional non-toxic salts of the parent compound formed, for example, from non-toxic inorganic or organic acids. The pharmaceutically acceptable salts of the compounds provided herein can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. The phrase "pharmaceutically acceptable salt" is not limited to a mono, or 1:1, salt. For example, "pharmaceutically acceptable salt" also includes bis-salts, such as a bis-hydrochloride salt. Lists of suitable salts are found in Remington's Pharmaceutical Sciences, 17th ed., Mack Publishing Company, Easton, Pa., 1985, p. 1418 and Journal of Pharmaceutical Science, 66, 2 (1977), each of which is incorporated herein by reference in its entirety.

As used herein, the term "composition" or "pharmaceutical composition" refers to a mixture of at least one compound provided herein with a pharmaceutically acceptable carrier. The pharmaceutical composition facilitates administration of the compound to a patient or subject. Multiple techniques of administering a compound exist in the art including, but not limited to, intravenous, oral, aerosol, parenteral, ophthalmic, pulmonary, and topical administration.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a provided herein within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound provided herein, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations.

As used herein, "pharmaceutically acceptable carrier" also includes any and all coatings, antibacterial and antifungal agents, and absorption delaying agents, and the like that are compatible with the activity of the compound provided herein, and are physiologically acceptable to the patient. Supplementary active compounds may also be incorporated into the compositions. The "pharmaceutically acceptable carrier" may further include a pharmaceutically acceptable salt of a compound provided herein. Other additional ingredients that may be included in the pharmaceutical compositions provided herein are known in the art and described, for example, in Remington's Pharmaceutical Sciences (Genaro, Ed., Mack Publishing Co., 1985, Easton, PA), which is incorporated herein by reference.

As used herein, the term "prodrug" refers to a biologically inactive compound, which can be metabolized in the body to produce the corresponding biologically active drug substance. A prodrug is a medication or compound that, after administration, is metabolized into a pharmacologically active drug.

The compounds disclosed herein may also exist in unsolvated and solvated forms. The term "solvate" is used herein to describe a molecular complex comprising the compound of the invention and one or more pharmaceutically acceptable solvent molecules, for example, ethanol.

As used herein, the term "alkyl," by itself or as part of another substituent means, unless otherwise stated, a straight or branched chain hydrocarbon having the number of carbon atoms designated (*i.e.,* C₁-C₆-alkyl means an alkyl having one to six carbon atoms) and includes straight and branched chains. In an embodiment, C₁-C₆ alkyl groups are provided herein. Examples include methyl, ethyl, propyl, isopropyl, butyl, isobutyl, tert-butyl, pentyl, neopentyl, and hexyl. Other examples of C₁-C₆-alkyl include ethyl, methyl, isopropyl, isobutyl, n-pentyl, and n-hexyl.

As used herein, the term "alkoxy" refers to an alkyl (carbon and hydrogen chain) group singular bonded to oxygenlike for instance a methoxy group or ethoxy group.

As used herein, the term "amino" refers to a functional group having the formulae -NH₂, -NH(alkyl), and -N(alkyl)₂, wherein alkyl is as defined herein.

As used herein, the term "amide" refers to a functional group having the formulae -C(O)N(R)₂ or -N(R)C(O)alkyl, wherein the carbon atom is doubly bound to the oxygen atom and R is independently at each occurrence hydrogen or alkyl.

As used herein, the term "ester" refers to a functional group having the formulae -C(O)alkoxy, CO₂alkyl, -OC(O)alkyl, wherein the carbon atom is doubly bound to one oxygen atom and singly bound to an alkoxy group as defined herein.

As used herein, the term "halo" or "halogen" alone or as part of another substituent means, unless otherwise stated, a fluorine, chlorine, bromine, or iodine atom, preferably, fluorine, chlorine, or bromine, more preferably, fluorine or chlorine.

As used herein, the term "nitrile" refers to the functional group -CN, where carbon is triply bound to nitrogen.

As used herein, the term "heterocycle" refers to molecules that are saturated or partially saturated an include tetrahydrofuran, oxetane, dioxane or other cyclic ethers. Heterocycle also includes bicyclic structures that may be bridged or spirocyclic in nature with each individual ring within the bicycle varying from 3-8 atoms, and containing 0, 1, or 2 N, O, or S atoms. The term "heterocyclyl" includes cyclic esters (i.e., lactones) and cyclic amides (i.e., lactams) and also specifically includes, but is not limited to, epoxidyl, oxetanyl, tetrahydrofuranyl, tetrahydropyranyl (i.e., oxanyl), pyranyl, dioxanyl, aziridinyl, azetidinyl, pyrrolidinyl, 2,5-dihydro-1H-pyrrolyl, oxazolidinyl, thiazolidinyl, piperidinyl, morpholinyl, piperazinyl, thiomorpholinyl, 1,3-oxazinanyl, 1,3-thiazinanyl, 2-azabicyclo[2.1.1]hexanyl, 5-azabicyclo[2.1.1]hexanyl, 6-azabicyclo[3.1.1] heptanyl, 2-azabicyclo[2.2.1]heptanyl, 3-azabicyclo[3.1.1]heptanyl, 2-azabicyclo[3.1.1]heptanyl, 3-azabicyclo[3.1.0]hexanyl, 2-azabicyclo[3.1.0]hexanyl, 3-azabicyclo[3.2.1]octanyl, 8-azabicyclo[3.2.1]octanyl, 3-oxa-7-azabicyclo[3.3.1]nonanyl, 3-oxa-9-azabicyclo[3.3.1]nonanyl, 2-oxa-5-azabicyclo[2.2.1]heptanyl, 6-oxa-3-azabicyclo[3.1.1]heptanyl, 2-azaspiro[3.3]heptanyl, 2-oxa-6-azaspiro[3.3]heptanyl, 2-oxaspiro[3.3]heptanyl, 2-oxaspiro[3.5]nonanyl, 3-oxaspiro[5.3]nonanyl, and 8-oxabicyclo[3.2.1]octanyl.

It will be understood that when a carbon is in the (R)-configuration, this implies that said carbon is an asymmetric carbon. As the skilled person will acknowledge, symmetric carbons, not being stereocenters, cannot be in the (*R*)- or (*S*)-configuration. Only asymmetric carbons can be in said configurations. Thus, it will be understood that the carbon of R³ bonded to the amine in the 4-position of the quinazoline is an asymmetric carbon.

### Compounds

In an aspect, provided herein is a compound of Formula I: 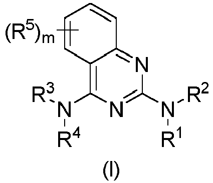 or a pharmaceutically acceptable salt thereof;
wherein
R¹ is selected from the group consisting of hydrogen, C₁-C₃ alkyl, -C(O)C₁-C₃ alkyl, - C(O)OC₁-C₃ alkyl, and -P(O)(OC₁-C₃ alkyl)₂;
R² is selected from the group consisting of C₁-C₃ alkyl, -C(O)C₁-C₃ alkyl, -C(O)OC₁-C₃ alkyl, -P(O)(OC₁-C₃ alkyl)₂, -(CH₂)₁₋₃C(O)C₁-C₃ alkyl, -(CH₂)₁₋₃C(O)OC₁-C₃ alkyl, -(CH₂)₁₋₃P(O)(OC₁-C₃ alkyl)₂ and C₃₋₆heterocycle comprising one or more heteroatom(s), the one or more heteroatom(s) being selected from oxygen; nitrogen and sulfur, more particularly oxygen
R³ is C₁-C₈ alkyl, wherein C₁-C₈ alkyl is optionally substituted by one or more substituents selected from halogen, -OH, -NH₂, amino, nitrile, ester, amide, C₁₋₃ alkyl and C₁₋₃ alkoxy;
the carbon of R³ bonded to the amine in the 4-position of the quinazoline is in (*R*)-configuration,
R⁴ is selected from the group consisting of hydrogen, C₁-C₈ alkyl, -C(O)C₁-C₃ alkyl, and - C(O)OC₁-C₃ alkyl;
R⁵ is independently, at each occurrence, selected from the group consisting of hydrogen, C₁-C₃ alkyl, -OC₁-C₃ alkyl, and halogen;
with the proviso that not all 4 occurrences of R⁵ are hydrogen; and
m is 4.

In an embodiment, the compound of Formula I is a compound of Formula II: 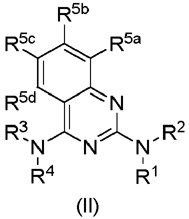 or a pharmaceutically acceptable salt thereof;
wherein
R¹ is selected from the group consisting of hydrogen, C₁-C₃ alkyl, -C(O)C₁-C₃ alkyl, - C(O)OC₁-C₃ alkyl, and -P(O)(OC₁-C₃ alkyl)₂;
R² is selected from the group consisting of C₁-C₃ alkyl, -C(O)C₁-C₃ alkyl, -C(O)OC₁-C₃ alkyl, -P(O)(OC₁-C₃ alkyl)₂, -(CH₂)₁₋₃C(O)C₁-C₃ alkyl, -(CH₂)₁₋₃C(O)OC₁-C₃ alkyl, -(CH₂)₁₋₃P(O)(OC₁-C₃ alkyl)₂ and C₃₋₆heterocycle comprising one or more heteroatom(s), the one or more heteroatom(s) being selected from oxygen; nitrogen and sulfur, more particularly oxygen;
R³ is C₁-C₈ alkyl, wherein C₁-C₈ alkyl is optionally substituted by one or more substituents selected from halogen, -OH, -NH₂, amino, nitrile, ester, amide, C₁₋₃ alkyl and C₁₋₃ alkoxy;
the carbon of R³ bonded to the amine in the 4-position of the quinazoline is in (*R*)-configuration,
R⁴ is selected from the group consisting of hydrogen, C₁-C₈ alkyl, -C(O)C₁-C₃ alkyl, and -C(O)OC₁-C₃ alkyl; and
R^{5a,} R^{5b}, R^{5c} and R^{5d} are independently selected from the group consisting of hydrogen, C₁-C₃ alkyl, -OC₁-C₃ alkyl, and halogen;
with the proviso that at least one of R^{5a,} R^{5b}, R^{5c} and R^{5d} is not hydrogen.

In an embodiment, R³ is of formula (f-1), (f-2), (f-3) or (f-4): 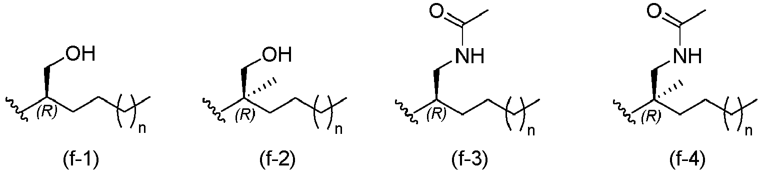 wherein n is 0, 1 or 2, more particularly 1.

In an embodiment, R² is selected from the group consisting of C₁-C₃ alkyl, -C(O)C₁-C₃ alkyl, -C(O)OC₁-C₃ alkyl, and -P(O)(OC₁-C₃ alkyl)₂.

In another embodiment, R¹ is hydrogen; R² is selected from the group consisting of -C(O)C₁-C₃ alkyl, -C(O)OC₁-C₃ alkyl, and -P(O)(OC₁-C₃ alkyl)₂; R³ is C₁-C₈ alkyl, wherein C₁-C₈ alkyl is optionally substituted with -OH, -NH₂, amide or halo; R⁴ is hydrogen; and R⁵ is independently, at each occurrence, C₁-C₃ alkyl or -OC₁-C₃ alkyl.

In yet another embodiment, R¹ is hydrogen; R² is selected from the group consisting of -C(O)C₁-C₃ alkyl, -C(O)OC₁-C₃ alkyl, and -P(O)(OC₁-C₃ alkyl)₂; R³ is C₁-C₈ alkyl, wherein C₁-C₈ alkyl is optionally substituted with -OH, -NH₂, or halogen; R⁴ is hydrogen; R⁵ is independently, at each occurrence, C₁-C₃ alkyl or -OC₁-C₃ alkyl; and m is 2.

In an embodiment, R² is -C(O)C₁-C₃ alkyl. In another embodiment, R² is -C(O)OC₁-C₃ alkyl.

In another embodiment, R³ is C₁-C₈ alkyl is optionally substituted with an ester, wherein the ester is -OC(O)C₁-C₄ alkyl. In yet another embodiment, R³ is C₁-C₈ alkyl substituted with amide, wherein amide is the formula -NHC(O)CH_{3.̅}

In an embodiment of Formula I, m is 1 and R⁵ is methyl. In an embodiment of Formula II, R^{5a} is methyl.

In another embodiment, R⁵ is independently, at each occurrence, selected from H and halogen (more particularly F).

In still another embodiment, R¹ is methyl. In an embodiment, R¹ is methyl and R⁴ is C₆ alkyl. In another embodiment, R¹ is methyl and R⁴ is C₆ alkyl substituted with -OH.

In still another embodiment, the compound of Formula I is a compound of Formula III: 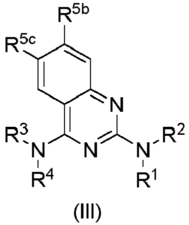 or a pharmaceutically acceptable salt thereof;
wherein R^{5b} and R^{5c} are independently, at each occurrence, selected from halogen (more particularly F) and H, with the proviso that at least one of R^{5b} and R^{5c} is not hydrogen; and R¹, R², R³ and R⁴ are as herein defined.

In another embodiment, the compound of Formula I is a compound of Formula IV: 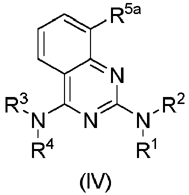 or a pharmaceutically acceptable salt thereof;
wherein R^{5a} is C₁₋₃ alkyl,
and R¹, R², R³ and R⁴ are as herein defined.

In an embodiment of Formula I, II, III or IV, R¹ is hydrogen. In another embodiment, R² is -C(O)C₁-C₃ alkyl. In yet another embodiment, R² is -C(O)OC₁-C₃ alkyl. In still another embodiment, R³ is C₁-C₆ alkyl. In an embodiment, R³ is C₁-C₆ alkyl substituted with -OH or ester. In another embodiment, R⁴ is hydrogen. In yet another embodiment, R² is -P(O)(OC₁-C₃ alkyl)₂ and R³ is C₁-C₅ alkyl substituted with -OH.

In another aspect, provided herein is a pharmaceutical composition comprising a compound disclosed herein, or a pharmaceutically acceptable salt thereof, and one of more pharmaceutically acceptable carriers or excipients.

In an embodiment, the application provides compound number 1 to compound number 2 as described in Table 1 below.

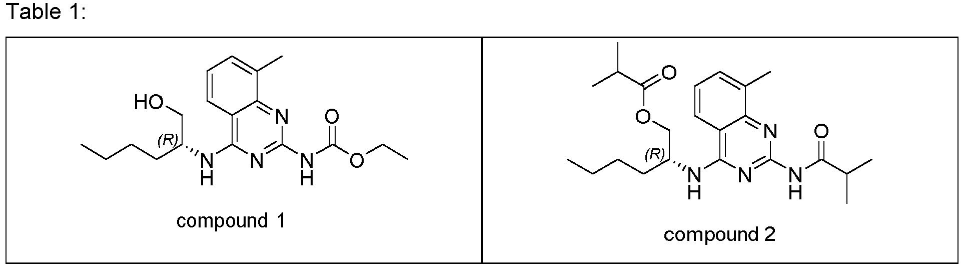

In one embodiment, the disclosed compounds may exist as tautomers. All tautomers are included within the scope of the compounds presented herein.

Compounds described herein also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described herein include and are not limited to ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P, and ³⁵S. In another embodiment, isotopically-labeled compounds are useful in drug or substrate tissue distribution studies. In another embodiment, substitution with heavier isotopes such as deuterium affords greater metabolic stability (for example, increased in vivo half-life or reduced dosage requirements). In yet another embodiment, the compounds described herein include a ²H (*i*.*e*., deuterium) isotope.

In still another embodiment, substitution with positron emitting isotopes, such as ¹¹C, ¹⁸F, ¹⁵O and ¹³N, is useful in Positron Emission Topography (PET) studies for examining substrate receptor occupancy. Isotopically-labeled compounds are prepared by any suitable method or by processes using an appropriate isotopically-labeled reagent in place of the non-labeled reagent otherwise employed.

The specific compounds described herein, and other compounds encompassed by one or more of the formulas described herein having different substituents are synthesized using techniques and materials described herein and as described, for example, in Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), Larock's Comprehensive Organic Transformations (VCH Publishers Inc., 1989), March, Advanced Organic Chemistry 4th Ed., (Wiley 1992); Carey and Sundberg, Advanced Organic Chemistry 4th Ed., Vols. A and B (Plenum 2000, 2001), and Green and Wuts, Protective Groups in Organic Synthesis 3rd Ed., (Wiley 1999) (all of which are incorporated by reference for such disclosure). General methods for the preparation of compounds as described herein are modified by the use of appropriate reagents and conditions, for the introduction of the various moieties found in the Formulas as provided herein.

In an embodiment, the compounds disclosed herein are considered prodrugs of pharmaceutically active ingredients. In another embodiment, the pharmaceutically active ingredient is characterized by bioavailability of 50% or less and a molecular weight in the range of 100-1000 Daltons. In yet another embodiment, the prodrug moiety is attached to the pharmaceutically active ingredient wherein the modification allows TLR8 agonist potential to be attenuated. In still another embodiment, the prodrug is enzymatically cleaved prior to or at the site of the liver to yield the pharmaceutically active ingredient, such that the pharmaceutically active ingredient is delivered to the individual limiting TLR8 agonism prior to the liver.

In an embodiment, the pharmaceutical compositions disclosed herein comprise at least one additional active or therapeutic agent. Additional active therapeutic agents may include, for example, an anti-HBV agent such as an HBV polymerase inhibitor, interferon, viral entry inhibitor, viral maturation inhibitor, capsid assembly modulator, reverse transcriptase inhibitor, immunomodulatory agent such as a TLR-agonist, or any other agents that affect the HBV life cycle and/or the consequences of HBV infection. The compounds disclosed herein may be used, alone or in combination with one or more additional active agents, to formulate a pharmaceutical composition.

Compounds described herein are synthesized using any suitable procedures starting from compounds that are available from commercial sources, or are prepared using procedures described herein.

### Uses

In an aspect, provided herein are compounds and pharmaceutical compositions comprising said compounds for use in the treatment or prevention of a viral infection in a subject in need thereof.

In an embodiment, the viral infection is a hepatitis B (HBV) infection. In another embodiment, a compound disclosed herein or a pharmaceutical composition thereof is administered as a vaccine adjuvant.

In another aspect, provided herein are compounds and pharmaceutical compositions comprising said compounds for use in the treatment of an immune disorder in a subject in need thereof.

In yet another aspect, provided herein are compounds and pharmaceutical compositions comprising said compounds for use in the treatment of cancer in a subject in need thereof.

In certain embodiments, the cancer is lung cancer, colon and rectal cancer, breast cancer, prostate cancer, liver cancer, pancreatic cancer, brain cancer, kidney cancer, ovarian cancer, stomach cancer, skin cancer, bone cancer, gastric cancer, breast cancer, glioma, glioblastoma, neuroblastoma, hepatocellular carcinoma, papillary renal carcinoma, head and neck squamous cell carcinoma, leukemia, lymphomas, myelomas, retinoblastoma, cervical cancer, melanoma and/or skin cancer, bladder cancer, uterine cancer, testicular cancer, esophageal cancer, and solid tumors. In some embodiments, the cancer is lung cancer, colon cancer, breast cancer, neuroblastoma, leukemia, or lymphomas. In other embodiments, the cancer is lung cancer, colon cancer, breast cancer, neuroblastoma, leukemia, or lymphoma. In a further embodiment, the cancer is non-small cell lung cancer (NSCLC) or small cell lung cancer.

In further embodiments, the cancer is a hematologic cancer. In an embodiment, the hematologic cancer is leukemia or lymphoma. In a certain embodiment, lymphoma is Hodgkin's lymphoma or Non-Hodgkin's lymphoma. In certain embodiments, leukemia is myeloid, lymphocytic, myelocytic, lymphoblastic, or megakaryotic leukemia.

In an embodiment, the compound or pharmaceutical composition comprising said compound induces a T helper 1 (Th1) response in the subject. In another embodiment, the induction of the Th1 response results in the secretion of IL-12p70 in the subject. In yet another embodiment, the induction of the Th1 response results in an upregulation of CD40 or OX40L in the subject. In still another embodiment, the induction of the Th1 response results in an upregulation of IFNγ in the subject.

In an embodiment, the compound disclosed herein or the pharmaceutical composition thereof is a Toll-like receptor (TLR) agonist. In another embodiment, the Toll-like receptor is Toll-like receptor 8 (TLR8). In yet another embodiment, the compound disclosed herein or pharmaceutical composition thereof is administered orally.

In a non-limiting aspect, these compounds disclosed herein may (i) modulate or disrupt HBV assembly and other HBV core protein functions necessary for HBV replication or the generation of infectious particles, (ii) inhibit the production of infectious virus particles or infection, or (iii) interact with HBV capsid to effect defective viral particles with reduced infectivity or replication capacity acting as capsid assembly modulators. In particular, and without being bound to any particular mechanism of action, it is believed that the disclosed compounds are useful in HBV treatment by disrupting, accelerating, reducing, delaying and/or inhibiting normal viral capsid assembly and/or disassembly of immature or mature particles, thereby inducing aberrant capsid morphology leading to antiviral effects such as disruption of virion assembly and/or disassembly, virion maturation, virus egress and/or infection of target cells. The disclosed compounds may act as a disruptor of capsid assembly interacting with mature or immature viral capsid to perturb the stability of the capsid, thus affecting its assembly and/or disassembly. The disclosed compounds may perturb protein folding and/or salt bridges required for stability, function and/or normal morphology of the viral capsid, thereby disrupting and/or accelerating capsid assembly and/or disassembly. The disclosed compounds may bind capsid and alter metabolism of cellular polyproteins and precursors, leading to abnormal accumulation of protein monomers and/or oligomers and/or abnormal particles, which causes cellular toxicity and death of infected cells. The disclosed compounds may cause failure of the formation of capsids of optimal stability, affecting efficient uncoating and/or disassembly of viruses (e.g., during infectivity). The disclosed compounds may disrupt and/or accelerate capsid assembly and/or disassembly when the capsid protein is immature. The disclosed compounds may disrupt and/or accelerate capsid assembly and/or disassembly when the capsid protein is mature. The disclosed compounds may disrupt and/or accelerate capsid assembly and/or disassembly during viral infectivity which may further attenuate HBV viral infectivity and/or reduce viral load. The disruption, acceleration, inhibition, delay and/or reduction of capsid assembly and/or disassembly by the disclosed compounds may eradicate the virus from the host organism. Eradication of HBV from a subject by the disclosed compounds advantageously obviates the need for chronic long-term therapy and/or reduces the duration of long-term therapy.

In another aspect, provided herein are compounds and pharmaceutical compositions comprising said compounds for use in reducing the viral load associated with an HBV infection in a subject in need thereof.

In another aspect, provided herein are compounds and pharmaceutical compositions comprising said compounds for use in reducing reoccurrence of an HBV infection in a subject in need thereof.

In another aspect, provided herein are compounds and pharmaceutical compositions comprising said compounds for use in inhibiting or reducing the formation or presence of HBV DNA-containing particles or HBV RNA-containing particles in a subject in need thereof.

In another aspect, provided herein are compounds and pharmaceutical compositions comprising said compounds for use in reducing an adverse physiological impact of an HBV infection in a subject in need thereof.

In another aspect, provided herein are compounds and pharmaceutical compositions comprising said compounds for use in inducing remission of hepatic injury from an HBV infection in a subject in need thereof.

In another aspect, provided herein are compounds and pharmaceutical compositions comprising said compounds for use in reducing the physiological impact of long-term antiviral therapy for HBV infection in a subject in need thereof.

In another aspect, provided herein are compounds and pharmaceutical compositions comprising said compounds for use in prophylactically treating an HBV infection in a subject in need thereof.

In an embodiment, the disclosed compounds are suitable for monotherapy. In another embodiment, the disclosed compounds are effective against natural or native HBV strains. In yet another embodiment, the disclosed compounds are effective against HBV strains resistant to currently known drugs.

In still another embodiment, the compounds provided herein can be used in modulating (e.g., inhibiting or disrupting) the activity, stability, function, and viral replication properties of HBV cccDNA.

In yet another embodiment, the compounds of the present disclosure can be used in diminishing or preventing the formation of HBV cccDNA.

In another embodiment, the compounds provided herein can be used in modulating (e.g., inhibiting or disrupting) the activity of HBV cccDNA.

In yet another embodiment, the compounds of the present disclosure can be used in diminishing the formation of HBV cccDNA.

In another embodiment, the disclosed compounds can be used in modulating, inhibiting, or disrupting the generation or release of HBV RNA particles from within the infected cell.

In a further embodiment, the total burden (or concentration) of HBV RNA particles is modulated. In a preferred embodiment, the total burden of HBV RNA is diminished.

In another embodiment, the compounds or pharmaceutical compositions of said compounds provided herein reduce the viral load in the subject to a greater extent or at a faster rate compared to the administering of a compound selected from the group consisting of an HBV polymerase inhibitor, interferon, viral entry inhibitor, viral maturation inhibitor, distinct capsid assembly modulator, antiviral compounds of distinct or unknown mechanism, and any combination thereof.

In another embodiment, the compounds or pharmaceutical compositions of said compounds provided herein cause a lower incidence of viral mutation and/or viral resistance than the administering of a compound selected from the group consisting of an HBV polymerase inhibitor, interferon, viral entry inhibitor, viral maturation inhibitor, distinct capsid assembly modulator, antiviral compounds of distinct or unknown mechanism, and combination thereof.

In another embodiment, the methods provided herein further comprise administering to the subject at least one HBV vaccine, a nucleoside HBV inhibitor, an interferon or any combination thereof.

In an embodiment of the methods, the subject is human.

### Combinations

Provided herein are combinations of one or more of the disclosed compounds with at least one additional therapeutic agent. In an embodiment, the methods provided herein can further comprise administering to the individual at least one additional therapeutic agent. In another embodiment, the disclosed compounds are suitable for use in combination therapy. The compounds disclosed herein may be useful in combination with one or more additional compounds useful for treating HBV infection. These additional compounds may comprise compounds of the present disclosure or compounds known to treat, prevent, or reduce the symptoms or effects of HBV infection.

In an embodiment, additional active ingredients are those that are known or discovered to be effective in the treatment of conditions or disorders involved in HBV infection, such as another HBV capsid assembly modulator or a compound active against another target associated with the particular condition or disorder involved in HBV infection, or the HBV infection itself. The combination may serve to increase efficacy (e.g., by including in the combination a compound potentiating the potency or effectiveness of an active agent according to the present disclosure), decrease one or more side effects, or decrease the required dose of the active agent according to the present disclosure. In another embodiment, the methods provided herein allow for administering of the at least one additional therapeutic agent at a lower dose or frequency as compared to the administering of the at least one additional therapeutic agent alone that is required to achieve similar results in prophylactically treating an HBV infection in an individual in need thereof.

Such compounds include, but are not limited to, HBV combination drugs, HBV vaccines, HBV DNA polymerase inhibitors, immunomodulatory agents, toll-like receptor (TLR) modulators, interferon alpha receptor ligands, hyaluronidase inhibitors, hepatitis b surface antigen (HBsAg) inhibitors, cytotoxic T-lymphocyte-associated protein 4 (ipi4) inhibitors, cyclophilin inhibitors, HBV viral entry inhibitors, antisense oligonucleotide targeting viral mRNA, short interfering RNAs (siRNA) and ddRNAi endonuclease modulators, ribonucleotide reductase inhibitors, HBV E antigen inhibitors, covalently closed circular DNA (cccDNA) inhibitors, famesoid X receptor agonists, HBV antibodies, CCR2 chemokine antagonists, thymosin agonists, cytokines, nucleoprotein modulators, retinoic acid-inducible gene 1 simulators, NOD2 stimulators, phosphatidylinositol 3-kinase (PI3K) inhibitors, indoleamine-2, 3-dioxygenase (IDO) pathway inhibitors, PD-1 inhibitors, PD-L1 inhibitors, recombinant thymosin alpha-1, bruton's tyrosine kinase (BTK) inhibitors, KDM inhibitors, HBV replication inhibitors, arginase inhibitors, and any other agent that affects the HBV life cycle and/or affect the consequences of HBV infection or combinations thereof.

In an embodiment, the compounds provided herein may be used in combination with an HBV polymerase inhibitor, immunomodulatory agents, interferon such as pegylated interferon, viral entry inhibitor, viral maturation inhibitor, capsid assembly modulator, reverse transcriptase inhibitor, a cyclophilin/TNF inhibitor, immunomodulatory agent such as a TLR-agonist, an HBV vaccine, and any other agent that affects the HBV life cycle and/or affect the consequences of HBV infection or combinations thereof.

### Administration /Dosage /Formulations

In an aspect, provided herein is a pharmaceutical composition comprising at least one compound provided herein, together with a pharmaceutically acceptable carrier.

The compounds disclosed herein may be administered as crystalline or amorphous products. They may be obtained, for example, as solid plugs, powders, or films by methods such as precipitation, crystallization, freeze drying, spray drying, or evaporative drying. They may be administered alone or in combination with one or more other compounds of the invention or in combination with one or more other drugs. Generally, they will be administered as a formulation in association with one or more pharmaceutically acceptable excipients.

Actual dosage levels of the active ingredients in the pharmaceutical compositions provided herein may be varied so as to obtain an amount of the active ingredient that is effective to achieve the desired therapeutic response for a particular patient, composition, and mode of administration, without being toxic to the patient.

In particular, the selected dosage level will depend upon a variety of factors including the activity of the particular compound employed, the time of administration, the rate of excretion of the compound, the duration of the treatment, other drugs, compounds or materials used in combination with the compound, the age, sex, weight, condition, general health and prior medical history of the patient being treated, and like factors well, known in the medical arts.

A medical doctor, e.g., physician or veterinarian, having ordinary skill in the art may readily determine and prescribe the effective amount of the pharmaceutical composition required. For example, the physician or veterinarian could begin administration of the pharmaceutical composition to dose the disclosed compound at levels lower than that required in order to achieve the desired therapeutic effect and gradually increase the dosage until the desired effect is achieved.

An example of a dose of a compound is from about 1 mg to about 2,500 mg. In some embodiments, a dose of a compound of the present disclosure used in compositions described herein is less than about 10,000 mg, or less than about 8,000 mg, or less than about 6,000 mg, or less than about 5,000 mg, or less than about 3,000 mg, or less than about 2,000 mg, or less than about 1,000 mg, or less than about 500 mg, or less than about 200 mg, or less than about 50 mg. Similarly, in some embodiments, a dose of a second compound (i.e., another drug for HBV treatment) as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof.

Once improvement of the patient's disease, disorder, or condition has occurred, the dose may be adjusted for preventative or maintenance treatment. For example, the dosage or the frequency of administration, or both, may be reduced as a function of the symptoms, to a level at which the desired therapeutic or prophylactic effect is maintained. Of course, if symptoms have been alleviated to an appropriate level, treatment may cease. Patients may, however, require intermittent treatment on a long-term basis upon any recurrence of symptoms. HBV infections that may be treated according to the disclosed methods include HBV genotype A, B, C, and/or D infections. However, in an embodiment, the methods disclosed may treat any HBV genotype ("pan-genotypic treatment"). HBV genotyping may be performed using methods known in the art, for example, INNO-LIPA^{®} HBV Genotyping, Innogenetics N.V., Ghent, Belgium).

In particular embodiments, it is especially advantageous to formulate the compound in dosage unit form for ease of administration and uniformity of dosage. Dosage unit form as used herein refers to physically discrete units suited as unitary dosages for the patients to be treated; each unit containing a predetermined quantity of the disclosed compound calculated to produce the desired therapeutic effect in association with the required pharmaceutical vehicle. The dosage unit forms of the compounds provided herein are dictated by and directly dependent on (a) the unique characteristics of the disclosed compound and the particular therapeutic effect to be achieved, and (b) the limitations inherent in the art of compounding/formulating such a disclosed compound for the treatment of pain, a depressive disorder, or drug addiction in a patient.

In one embodiment, the compounds provided herein are formulated using one or more pharmaceutically acceptable excipients or carriers. In one embodiment, the pharmaceutical compositions provided herein comprise a therapeutically effective amount of a disclosed compound and a pharmaceutically acceptable carrier.

Routes of administration of any of the compositions provided herein include oral, nasal, rectal, intravaginal, parenteral, buccal, sublingual or topical. The compounds provided herein may be formulated for administration by any suitable route, such as for oral or parenteral, for example, transdermal, transmucosal (e.g., sublingual, lingual, (trans)buccal, (trans)urethral, vaginal (e.g., trans- and perivaginally), (intra)nasal and (trans)rectal), intravesical, intrapulmonary, intraduodenal, intragastrical, intrathecal, subcutaneous, intramuscular, intradermal, intra-arterial, intravenous, intrabronchial, inhalation, and topical administration. In one embodiment, the preferred route of administration is oral. Suitable compositions and dosage forms include, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful are not limited to the particular formulations and compositions that are described herein.

For oral application, particularly suitable are tablets, dragees, liquids, drops, suppositories, or capsules, caplets and gel caps. The compositions intended for oral use may be prepared according to any method known in the art and such compositions may contain one or more agents selected from the group consisting of inert, non-toxic pharmaceutically excipients that are suitable for the manufacture of tablets. Such excipients include, for example an inert diluent such as lactose; granulating and disintegrating agents such as cornstarch; binding agents such as starch; and lubricating agents such as magnesium stearate. The tablets may be uncoated or they may be coated by known techniques for elegance or to delay the release of the active ingredients. Formulations for oral use may also be presented as hard gelatin capsules wherein the active ingredient is mixed with an inert diluent.

For parenteral administration, the disclosed compounds may be formulated for injection or infusion, for example, intravenous, intramuscular or subcutaneous injection or infusion, or for administration in a bolus dose or continuous infusion. Suspensions, solutions or emulsions in an oily or aqueous vehicle, optionally containing other formulatory agents such as suspending, stabilizing or dispersing agents may be used. Those skilled in the art will recognize, or be able to ascertain using no more than routine experimentation, numerous equivalents to the specific procedures, embodiments, claims, and examples described herein. Such equivalents were considered to be within the scope of the examples provided herein and covered by the claims appended hereto. For example, it should be understood, that modifications in reaction conditions, including but not limited to reaction times, reaction size/volume, and experimental reagents, such as solvents, catalysts, pressures, atmospheric conditions, e.g., nitrogen atmosphere, and reducing/oxidizing agents, with art-recognized alternatives and using no more than routine experimentation, are within the scope of the present application.

It is to be understood that wherever values and ranges are provided herein, all values and ranges encompassed by these values and ranges, are meant to be encompassed within the scope of the examples provided herein. Moreover, all values that fall within these ranges, as well as the upper or lower limits of a range of values, are also contemplated by the present application.

The following examples further illustrate aspects of the compounds provided herein. However, they are in no way a limitation of the teachings or disclosure presented herein.

### EXAMPLES

The compounds provided herein are further illustrated by the following examples, which should not be construed as further limiting. The methods of preparing and using the compounds provided herein will employ, unless otherwise indicated, conventional techniques of organic synthesis, cell biology, cell culture, molecular biology, transgenic biology, microbiology and immunology, which are within the skill of the art.

### Experimental section

### Preparation of 2-amino-8-methylquinazolin-4-ol (A1)

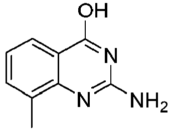

Into a 250 mL round bottom flask equipped with a magnetic stir bar was placed 2-amino-3-methylbenzoic acid (10 g, 66.15 mmol), EtOH (250 mL), cyanamide (4.17 g, 99.2 mmol), and concentrated HCl (3 mL). The mixture stirred at reflux for 6h. At 1h intervals, concentrated HCl (0.5 mL) was added via pipette. The reaction mixture cooled to rt and the solids were isolated via filtration and washed with EtOH and dried under vacuum to afford the titled compound as an off-white solid (4.8 g). ¹H NMR (400 MHz, DMSO-*d₆*) δ ppm 2.41 (s, 3 H), 7.15 (t, J=7.5 Hz, 1 H), 7.43 (br. s., 2 H), 7.55 (d, J=7.0 Hz, 1 H), 7.80 (d, J=7.8 Hz, 1 H), 11.17 - 12.49 (m, 1 H). MS (ESI) *m*/*z* = 176 [M+H].

Preparation of (R)-2-((2-amino-8-methylquinazolin-4-yl)amino)pentan-1-ol (A2)

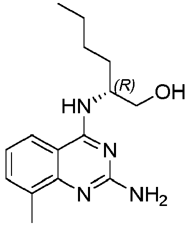

Into a 50 mL glass vial was placed 2-amino-8-methylquinazolin-4-ol (500 mg, 2.71 mmol), anhydrous DMF (10 mL), DBU (1.22 mL, 8.13 mmol), and D-norvalinol (1.40 g, 13.6 mmol). To this solution was added BOP (1.44 g, 3.3 mmol). The vial was sealed and shaken for 15h at rt. The solvent was removed under reduced pressure. NaOH (1M, aq., 10 mL) was added and washed with EtOAc (5 × 20 mL). The organic layers were combined, dried (MgSO4), the solids were removed by filtration, and the solvents of the filtrate were removed under reduced pressure. EtOAc was added to the mixture, the product precipitated and was isolated as a white solid (309 mg). MS (ESI) *m*/*z* = 275 [M+H]. ¹H NMR (400 MHz, DMSO-*d*₆) δ ppm 0.81 - 0.90 (m, 3 H), 1.20 - 1.37 (m, 4 H), 1.49 - 1.61 (m, 1 H), 1.64 - 1.76 (m, 1 H), 2.37 (s, 3 H), 3.41 - 3.55 (m, 2 H), 4.34 (td, J=8.7, 5.3 Hz, 1 H), 4.66 (t, J=5.5 Hz, 1 H), 5.88 (s, 2 H), 6.90 (dd, J=8.0, 7.2 Hz, 1 H), 7.17 (d, J=8.4 Hz, 1 H), 7.33 (d, J=7.0 Hz, 1 H), 7.88 (d, J=7.9 Hz, 1 H).

### Preparation of ethyl (4-hydroxy-8-methylquinazolin-2-yl)carbamate (A3)

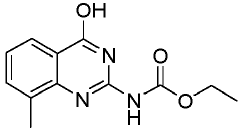

Ethoxycarbonylisothiocyanate (1.57 g, 12 mmol) was added to a solution of methyl 2-amino-3-methylbenzoate (1.65 g, 10 mmol) in acetonitrile (50 mL) under a nitrogen atmosphere at room temperature. Stirring continued overnight at ambient temperature. Then HMDS (21.24 mL, 100 mmol) and EDCI (3.83 g, 20 mmol) were added to the reaction mixture and stirring was continued overnight at room temperature. The solvents were removed under reduced pressure and the crude was stirred in water for two hours. The white precipitate was collected by filtration and dried in vacuo to afford the titled compound (2.47g). ¹H NMR (360 MHz, DMSO-*d*₆) δ ppm 1.27 (t, J=7.1 Hz, 3 H), 2.45 (s, 3 H), 4.23 (q, J=7.2 Hz, 2 H), 7.26 (t, J=7.7 Hz, 1 H), 7.61 (d, J=7.3 Hz, 1 H), 7.88 (d, J=8.1 Hz, 1 H), 11.20 (br s, 1 H), 11.48 (br s, 1 H).

Preparation of ethyl (R)-(4-((1-hydroxyhexan-2-yl)amino)-8-methylquinazolin-2-yl)carbamate (1)

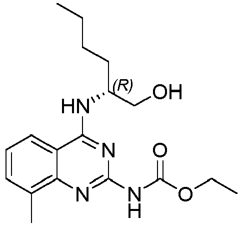

A solution of ethyl (4-hydroxy-8-methylquinazolin-2-yl)carbamate (494.51 mg, 2 mmol), DBU (0.6 mL, 4 mmol) in anhydrous DMF (10 mL) was stirred at room temperature under a nitrogen atmosphere. BOP (973 mg, 11 mmol) was added portion wise and stirring continued for 15 minutes. D-norleucinol (0.47 g, 4 mmol) was added and stirring continued for 2h. The mixture was poured into ice water while stirring was continued for 1h. The water layer was extracted with ethyl acetate (3x), the combined organic layers were once washed with water and brine. The organic phase was dried over MgSO₄, filtered and evaporated. The corresponding fractions were evaporated and dried in vacuo to afford the titled compound (599 mg). ¹H NMR (360 MHz, DMSO-*d*₆) δ ppm 0.81 - 0.87 (m, 3 H), 1.19 - 1.38 (m, 7 H), 1.50 - 1.77 (m, 2 H), 2.42 - 2.49 (m, 3 H), 3.52 (t, J=5.7 Hz, 2 H), 4.11 (q, J=7.0 Hz, 2 H), 4.33 - 4.48 (m, 1 H), 4.66 (t, J=5.5 Hz, 1 H), 7.19 (t, J=7.7 Hz, 1 H), 7.51 (d, J=7.3 Hz, 1 H), 7.65 (br d, J=8.1 Hz, 1 H), 8.07 (d, J=8.1 Hz, 1 H), 9.52 (s, 1 H).

### Preparation of (R)-2-((2-isobutyramido-8-methylquinazolin-4-yl)amino)hexyl isobutyrate (2)

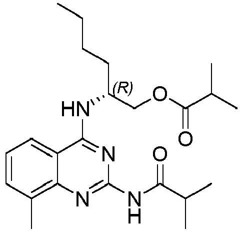

(R)-2-((2-amino-8-methylquinazolin-4-yl)amino)hexan-1-ol (2.1 g, 7.65 mmol) was dissolved in CH₂Cl₂ (40 mL) and cooled to 0°C. DBU (2.3 mL, 15.3 mmol) was added and the mixture was stirred 30 minutes. Isobutyryl chloride (1.6 mL, 15.3 mmol) in CH₂Cl₂ (10 mL) was added dropwise and the mixture was stirred at room temperature for 18h. The mixture was diluted with CH₂Cl₂ and washed with water (2x). The organic layer was dried over MgSO₄, the solids were removed by filtration and the solvent of the filtrate was removed under reduced pressure. The crude was purified via silica column chromatography using CH₂Cl₂ / CH₃OH: 100/0 to 95/5 as a gradient. The corresponding fractions were evaporated and dried in vacuo. The crude was further purified via preparatory HPLC (Stationary phase: RP XBridge C18 OBD-10µm, 50 × 150 mm, mobile phase: 0.25% NH₄HCO₃ solution in H₂O, CH₃CN) yielding the titled compound as a white solid (1015 mg). ¹H NMR (360 MHz, DMSO-*d*₆) δ ppm 0.78 - 0.88 (m, 3 H), 0.88 - 0.90 (m, 1 H), 0.97 (dd, J=7.0, 2.6 Hz, 6 H), 1.11 (dd, J=7.0, 1.8 Hz, 6 H), 1.23 - 1.38 (m, 4 H), 1.65 (br d, J=6.2 Hz, 2 H), 2.31 - 2.50 (m, 4 H), 3.38 - 3.46 (m, 1 H), 4.03 (q, J=7.1 Hz, 1 H), 4.08 - 4.32 (m, 2 H), 4.57 - 4.77 (m, 1 H), 7.23 (t, J=7.5 Hz, 1 H), 7.54 (d, J=7.0 Hz, 1 H), 7.85 (d, J=8.4 Hz, 1 H), 8.05 (d, J=8.4 Hz, 1 H), 9.61 (s, 1 H).

### Measurement of TLR7 and TLR8 activity

The ability of compounds to activate human TLR7 and/or TLR8 was assessed in a cellular reporter assay using HEK293 cells transiently transfected with a TLR7 or TLR8 expression vector and NFκB-luc reporter construct. In one instance, the TLR expression construct expresses the respective wild type sequence or a mutant sequence comprising a deletion in the second leucine-rich repeat of the TLR. Such mutant TLR proteins have previously been shown to be more susceptible to agonist activation (US 7,498,409 in the name of Schering Corporation, the content of which is herein incorporated by reference).

HEK293 cells were grown in culture medium (DMEM supplemented with 10% FCS and 2 mM Glutamine). For transfection of cells in 10 cm dishes, cells were detached with Trypsin-EDTA, transfected with a mix of CMV-TLR7 or TLR8 plasmid (750 ng), NFκB-luc plasmid (375 ng) and a transfection reagent and incubated 24h at 37°C in a humidified 5% CO₂ atmosphere. Transfected cells were then detached with Trypsin-EDTA, washed in PBS and re-suspended in medium to a density of 1.67 × 10⁵ cells/mL. Thirty microliters of cells were then dispensed into each well in 384-well plates, where 10 µL of compound in 4% DMSO was already present. Following 6h incubation at 37°C, 5% CO₂, the luciferase activity was determined by adding 15 µl of STEADY LITE PLUS substrate (PERKIN ELMER) to each well and readout performed on a VIEWLUX ULTRAHTS microplate imager (PERKIN ELMER). Dose response curves were generated from measurements performed in quadruplicates. Lowest effective concentrations (LEC) values, defined as the concentration that induces an effect which is at least two-fold above the standard deviation of the assay, were determined for each compound.

Compound toxicity has been determined in parallel using a similar dilution series of compound with 30 µL per well of cells transfected with the CMV-TLR7 construct alone (1.67 × 10⁵ cells/mL), in 384-well plates. Cell viability has been measured after 6h incubation at 37°C, 5% CO₂ by adding 15µL of ATP lite (PERKIN ELMER) per well and reading on a ViewLux ultraHTS microplate imager (PERKIN ELMER). Data was reported as CC₅₀.

**Table 2: Biological activity of compounds of formula (I)**

| Compound | hTLR7 (LEC) | hTLR8 (LEC) |
|---|---|---|
| 1 | >25 | 17.5 |
| 2 | >25 | 6.4 |

### Absorption, Distribution, Metabolism, and Excretion (ADME) Data

**Intrinsic clearance (CLᵢₙₜ).** CLᵢₙₜ was determined in rat and human liver microsomes. Incubations were performed at 37°C, at a concentration of 1µM of compound, and a microsomal protein concentration of 1 mg/mL. Serial samples were removed at intervals of up to 60 min and analyzed for the concentration of compound to determine its intrinsic clearance rate. Compound was incubated in rat and human hepatocytes (10⁶ cells/mL) at 1 µM for 0, 10, 20, 40, 60 and 120 min. Serial samples were removed at intervals of up to 120 min and analyzed for the concentration of compound to determine its intrinsic clearance rate.

**Permeability and Efflux *in vitro.*** The *in vitro* permeability and potential to be transported by P-glycoprotein (P-gp) was determined using an MDCK cell line transfected with human MDR1 (P-glycoprotein). Compound was added to the apical (A) or basolateral (B) side of a confluent monolayer of MDCK-MDR1 cells. Permeability in the A→B direction in absence and presence of GF120918 and in the B→A direction in absence of GF120918 was measured by monitoring the appearance of the test compound on the opposite side of the membrane using a specific LC-MS/MS method. The efflux ratio (B→A -GF120918/A→B -GF120918) was calculated to determine whether the test compound was a P-gp substrate.

**Plasma stability.** Plasma stability is measured in rat and human plasma for 7 or 24 h at 37°C. Compounds are incubated at 1 µM. Incubations are performed manually in a 24-well plate and serial samples (100 µL) are removed at various time points prior to quenching with acetonitrile. Samples were analyzed by LC-MS/MS (without internal standard). Percentage remaining at each time point is calculated relative to the average peak area of the t=0 sample. Half-life (t_{1/2} in hours) is calculated from those data.

**SGF stability and FASSIF stability.** The stability of the prodrugs in simulated gastric fluid (SGF) in the presence of pepsin and in fasted simulated intestinal fluid (FASSIF) supplemented with pancreatine and esterase are measured. Test compounds are incubated *in vitro* for up to 2 hours and can be sampled at different time points. Samples are analyzed for parent compound disappearance by LC-MS/MS. Percentage remaining at each time point is calculated relative to the average peak area of the t=0 sample. Half-life (t_{1/2} in hours) is calculated from that data. **Pharmacokinetics in rat.** Prodrugs are administered orally to SD rats using, for example, aqueous solution at 5 mg/kg eq dose. Plasma samples are collected at different time points and the concentration of parent and prodrug are analyzed using LC-MS/MS. PK parameters can be calculated; Cₘₐₓ (ng/mL) and AUC₍₀₋ₗₐₛₜ₎ (ng.h/mL) for both prodrug and parent.

**Table 3: Permeability in MDCK-MDR1 cell line.**

| Entry | Papp A->B + GF120918 | Papp A->B - GF120918 | Efflux ratio B->A/A->B |
|---|---|---|---|
| **1** | 37.3 | 8.22 | 4.5 |
| **2** | NA | NA | NA |

| | | | |
|---|---|---|---|
| NA; not available. | | | |

**Table 4: Intrinsic clearance (CLint) in liver microsomes and hepatocytes.**

| Entry | CLᵢₙₜ Rat liver microsomes | CLᵢₙₜ Human liver microsomes | CLᵢₙₜ Hepatocytes rat | CLᵢₙₜ Hepatocytes human |
|---|---|---|---|---|
| **1** | 109 | 47 | NA | NA |
| **2** | 84 | NA | NA | NA |

| | | | | |
|---|---|---|---|---|
| NA; not available. | | | | |

## Claims

1. A compound of Formula I: 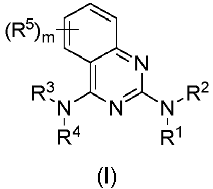 or a pharmaceutically acceptable salt thereof;
wherein
R¹ is selected from the group consisting of hydrogen, C₁-C₃ alkyl, -C(O)C₁-C₃ alkyl, -C(O)OC₁-C₃ alkyl, and -P(O)(OC₁-C₃ alkyl)₂;
R² is selected from the group consisting of C₁-C₃ alkyl, -C(O)C₁-C₃ alkyl, -C(O)OC₁-C₃ alkyl, -P(O)(OC₁-C₃ alkyl)₂, -(CH₂)₁₋₃C(O)C₁-C₃ alkyl, -(CH₂)₁₋₃C(O)OC₁-C₃ alkyl, -(CH₂)₁₋₃P(O)(OC₁-C₃ alkyl)₂ and C₃₋₆heterocycle comprising one or more heteroatom(s), the one or more heteroatom(s) being selected from oxygen, nitrogen and sulfur, more particularly oxygen;
R³ is C₁-C₈ alkyl, wherein C₁-C₈ alkyl is optionally substituted by one or more substituents selected from halogen, -OH, -NH₂, amino, nitrile, ester, amide, C₁₋₃ alkyl and C₁₋₃ alkoxy;
the carbon of R³ bonded to the amine in the 4-position of the quinazoline is in (R)-configuration,
R⁴ is selected from the group consisting of hydrogen, C₁-C₈ alkyl, -C(O)C₁-C₃ alkyl, and -C(O)OC₁-C₃ alkyl;
R⁵ is independently, at each occurrence, selected from the group consisting of hydrogen, C₁-C₃ alkyl, -OC₁-C₃ alkyl, and halogen;
with the proviso that not all 4 occurrences of R⁵ are hydrogen; and
m is 4.

2. The compound of claim 1, wherein R² is selected from the group consisting of C₁-C₃ alkyl, -C(O)C₁-C₃ alkyl, -C(O)OC₁-C₃ alkyl, and -P(O)(OC₁-C₃ alkyl)₂.

3. The compound of claim 1 or 2, wherein
R¹ is hydrogen;
R² is selected from the group consisting of -C(O)C₁-C₃ alkyl, -C(O)OC₁-C₃ alkyl, and - P(O)(OC₁-C₃ alkyl)₂;
R³ is C₁-C₈ alkyl, wherein C₁-C₈ alkyl is optionally substituted with -OH, -NH₂, or halo; R⁴ is hydrogen;
R⁵ is independently, at each occurrence, C₁-C₃ alkyl or -OC₁-C₃ alkyl; and
m is 2.

4. The compound of any one of claims 1-3, wherein R⁵ is independently, at each occurrence, selected from hydrogen and halogen, more particularly F, with the proviso that not all 4 occurrences of R⁵ are hydrogen, or
wherein the compound of Formula (I) is a compound of Formula III: 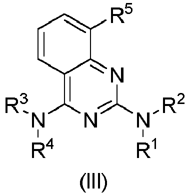 or a pharmaceutically acceptable salt thereof;
wherein R⁵ is C₁₋₃ alkyl.

5. The compound of any one of claims 1-4, wherein R¹ is hydrogen.

6. The compound of any one of claims 1-5, wherein R² is -C(O)C₁-C₃ alkyl or is -C(O)OC₁-C₃ alkyl.

7. The compound of any one of claims 1-6, wherein R³ is C₁-C₆ alkyl or is C₁-C₆ alkyl substituted with -OH or ester.

8. The compound of any one of claims 1-7, wherein R⁴ is hydrogen.

9. The compound of claim 1, which is 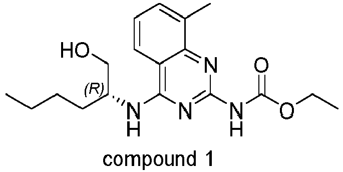 or

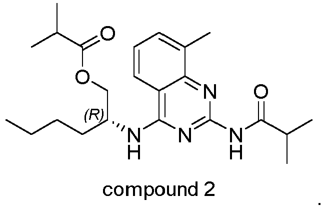

10. The compound of any one of claims 1-9, which is a Toll-like receptor (TLR) agonist, and wherein the Toll-like receptor optionally is Toll-like receptor 8 (TLR8).

11. A pharmaceutical composition comprising the compound of any one of claims 1-10, or a pharmaceutically acceptable salt thereof, and one of more pharmaceutically acceptable carriers or excipients.

12. The compound of any one of claims 1-10, or the pharmaceutical composition of claim 11, for use in the treatment or prevention of a viral infection in a subject in need thereof.

13. The compound of any one of claims 1-10, or the pharmaceutical composition of claim 11, for the use of claim 12, wherein the viral infection is a hepatitis B (HBV) infection.

14. The compound of any one of claims 1-10, or the pharmaceutical composition of claim 11, for the use of claim 12 or 13, wherein said compound or composition is administered as a vaccine adjuvant.

15. The compound of any one of claims 1-10, or the pharmaceutical composition of claim 11, for use in the treatment of an immune disorder in a subject in need thereof, or in the treatment of cancer.

16. The compound of any one of claims 1-10, or the pharmaceutical composition of claim 11, for the use of any one of claims 12-15, wherein said compound or composition induces a T helper 1 (Th1) response in the subject,
wherein the Th1 response in the subject optionally results in the secretion of IL-12p70 in the subject,
wherein the Th1 response optionally results in an upregulation of CD40 or OX40L in the subject, and
wherein the Th1 response optionally results in an upregulation of IFNγ in the subject.

17. The compound of any one of claims 1-10, or the pharmaceutical composition of claim 11, for the use of any one of claims 12-16, wherein said compound or pharmaceutical composition, is administered orally.

## Patentansprüche

1. Verbindung der Formel I: 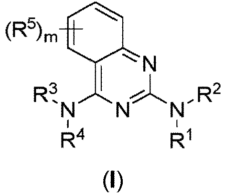 oder ein pharmazeutisch unbedenkliches Salz davon; wobei
R¹ aus der Gruppe bestehend aus Wasserstoff, C₁-C₃ Alkyl, -C(O)C₁-C₃ Alkyl, -C(O)OC₁-C₃ Alkyl und -P(0) (OC₁-C₃Alkyl)₂ ausgewählt ist;
R² aus der Gruppe bestehend aus C₁-C₃ Alkyl, -C(O)C₁-C₃ Alkyl, -C(O)OC₁-C₃ Alkyl, -P(O)(OC₁-C₃ Alkyl) ₂, -(CH₂)₁₋₃C(O)C₁-C₃ Alkyl, - (CH₂) ₁₋₃C(O)OC₁-C₃ Alkyl, - (CH₂)₁₋₃P(O)(OC₁-C₃ Alkyl) ₂ und C₃₋₆Heterocyclyl mit einem oder mehreren Heteroatomen, wobei das eine bzw. die mehreren Heteroatome aus Sauerstoff, Stickstoff und Schwefel, spezieller Sauerstoff, ausgewählt sind, ausgewählt ist;
R³ für C₁-C₈ Alkyl steht, wobei das C₁-C₈ Alkyl gegebenenfalls durch einen oder mehrere Substituenten, die aus Halogen, -OH, -NH₂, Amino, Nitril, Ester, Amid, C₁₋₃ Alkyl und C₁₋₃ Alkoxy ausgewählt sind, substituiert ist;
der Kohlenstoff von R³, der an das Amin in der 4-Position des Chinazolins gebunden ist, in der (R)-Konfiguration vorliegt,
R⁴ aus der Gruppe bestehend aus Wasserstoff, C₁-C₈ Alkyl, -C(O)C₁-C₃ Alkyl und -C(O)OC₁-C₃ Alkyl ausgewählt ist;
R⁵ unabhängig bei jedem Vorkommen aus der Gruppe bestehend aus Wasserstoff, C₁-C₃ Alkyl, -OC₁-C₃ Alkyl und Halogen ausgewählt ist;
mit der Maßgabe, dass nicht alle 4 Vorkommen von R⁵ Wasserstoff sind; und
m für 4 steht.

2. Verbindung nach Anspruch 1, wobei R² aus der Gruppe bestehend aus C₁-C₃ Alkyl, -C(O)C₁-C₃ Alkyl, -C(O)OC₁-C₃ Alkyl und -P(O)(OC₁-C₃ Alkyl) ₂ ausgewählt ist.

3. Verbindung nach Anspruch 1 oder 2, wobei R¹ für Wasserstoff steht;
R² aus der Gruppe bestehend aus -C(O)C₁-C₃ Alkyl, -C(O)OC₁-C₃ Alkyl und -P(O)(OC₁-C₃ Alkyl) ₂ ausgewählt ist;
R³ für C₁-C₈ Alkyl steht, wobei das C₁-C₈ Alkyl gegebenenfalls durch -OH, -NH₂ oder Halogen substituiert ist;
R⁴ für Wasserstoff steht;
R⁵ unabhängig bei jedem Vorkommen für C₁-C₃ Alkyl oder -OC₁-C₃ Alkyl steht; und
m für 2 steht.

4. Verbindung nach einem der Ansprüche 1-3, wobei R⁵ unabhängig bei jedem Vorkommen aus Wasserstoff und Halogen, spezieller F, ausgewählt ist, mit der Maßgabe, dass nicht alle 4 Vorkommen von R⁵ Wasserstoff sind, oder
wobei es sich bei der Verbindung der Formel (I) um eine Verbindung der Formel III: 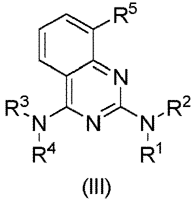 oder ein pharmazeutisch unbedenkliches Salz davon handelt;
wobei R⁵ für C₁-C₃-Alkyl steht.

5. Verbindung nach einem der Ansprüche 1-4, wobei R¹ für Wasserstoff steht.

6. Verbindung nach einem der Ansprüche 1-5, wobei R² für -C(O)C₁-C₃ Alkyl oder für -C(O)OC₁-C₃ Alkyl steht.

7. Verbindung nach einem der Ansprüche 1-6, wobei R³ für C₁-C₆ Alkyl oder für C₁-C₆ Alkyl, das durch -OH oder Ester substituiert ist, steht.

8. Verbindung nach einem der Ansprüche 1-7, wobei R⁴ für Wasserstoff steht.

9. Verbindung nach Anspruch 1, bei der es sich um 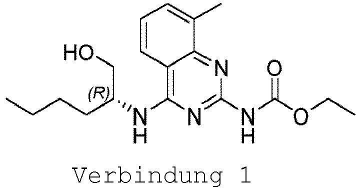 oder 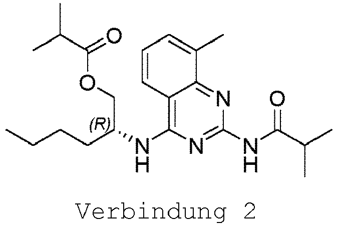 handelt.

10. Verbindung nach einem der Ansprüche 1-9, bei der es sich um einen Toll-Like-Receptor(TLR)-Agonisten handelt und wobei es sich bei dem Toll-Like-Receptor gegebenenfalls um Toll-Like-Receptor 8 (TLR8) handelt.

11. Pharmazeutische Zusammensetzung, umfassend die Verbindung nach einem der Ansprüche 1-10 oder ein pharmazeutisch unbedenkliches Salz davon und einen oder mehrere pharmazeutisch unbedenkliche Träger oder Hilfsstoffe.

12. Verbindung nach einem der Ansprüche 1-10 oder pharmazeutische Zusammensetzung nach Anspruch 11 zur Verwendung bei der Behandlung oder Prävention einer Virusinfektion bei einem Individuum, bei dem diesbezüglicher Bedarf besteht.

13. Verbindung nach einem der Ansprüche 1-10 oder pharmazeutische Zusammensetzung nach Anspruch 11 zur Verwendung nach Anspruch 12, wobei es sich bei der Virusinfektion um eine Hepatitis-B(HBV)-Infektion handelt.

14. Verbindung nach einem der Ansprüche 1-10 oder pharmazeutische Zusammensetzung nach Anspruch 11 zur Verwendung nach Anspruch 12 oder 13, wobei die Verbindung bzw. Zusammensetzung als Impfstoff-Adjuvans verabreicht wird.

15. Verbindung nach einem der Ansprüche 1-10 oder pharmazeutische Zusammensetzung nach Anspruch 11 zur Verwendung bei der Behandlung einer Immunstörung bei einem Individuum, bei dem diesbezüglicher Bedarf besteht, oder bei der Behandlung von Krebs.

16. Verbindung nach einem der Ansprüche 1-10 oder pharmazeutische Zusammensetzung nach Anspruch 11 zur Verwendung nach einem der Ansprüche 12-15, wobei die Verbindung bzw. Zusammensetzung eine T-Helfer-1(Th1)-Antwort in dem Individuum induziert,
wobei die Th1-Antwort in dem Individuum gegebenenfalls zur Sekretion von IL-12p70 in dem Individuum führt,
wobei die Th1-Antwort gegebenenfalls zu einer Hochregulierung von CD40 oder OX40L in dem Individuum führt und
wobei die Th1-Antwort gegebenenfalls zu einer Hochregulierung von IFNy in dem Individuum führt.

17. Verbindung nach einem der Ansprüche 1-10 oder pharmazeutische Zusammensetzung nach Anspruch 11 zur Verwendung nach einem der Ansprüche 12-16, wobei die Verbindung bzw. pharmazeutische Zusammensetzung oral verabreicht wird.

## Revendications

1. Composé de formule I : 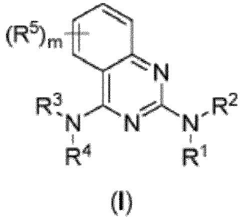 ou sel pharmaceutiquement acceptable correspondant ;
R¹ étant choisi dans le groupe constitué par hydrogène, C₁-C₃ alkyle, -C(O)C₁-C₃ alkyle, -C(O)OC₁-C₃ alkyle, et -P (0) (OC₁-C₃ alkyle) ₂ ;
R² étant choisi dans le groupe constitué par C₁-C₃ alkyle, -C(O)C₁-C₃ alkyle, -C(O)OC₁-C₃ alkyle, - P(O)(OC₁-C₃ alkyle)₂, -(CH₂)₁₋₃C(O)C₁-C₃ alkyle, - (CH₂)₁₋₃C(O)OC₁-C₃ alkyle, -(CH₂)₁₋₃P(O)(OC₁-C₃ alkyle)₂ et C₃₋₆hétérocycle comprenant un ou plusieurs hétéroatomes, l'hétéroatome ou les hétéroatomes étant choisis parmi oxygène, azote et soufre, plus particulièrement oxygène ;
R³ étant C₁-C₈ alkyle, C₁-C₈ alkyle étant éventuellement substitué par un ou plusieurs substituants choisis parmi halogène, -OH, -NH₂, amino, nitrile, ester, amide, C₁₋₃ alkyle et C₁-₃ alcoxy ;
le carbone de R³ lié à l'amine en position 4 de la quinazoline étant en configuration (*R*),
R⁴ étant choisi dans le groupe constitué par hydrogène, C₁-C₈ alkyle, -C(O)C₁-C₃ alkyle, et - C(O)OC₁-C₃ alkyle ;
R⁵ étant indépendamment en chaque occurrence choisi dans le groupe constitué par hydrogène, C₁-C₃ alkyle, -OC₁-C₃ alkyle, et halogène ;
à la condition que toutes les 4 occurrences de R⁵ ne soient pas hydrogène ; et
m étant 4.

2. Composé selon la revendication 1, R² étant choisi dans le groupe constitué par C₁-C₃ alkyle, -C(O)C₁-C₃ alkyle, -C(O)OC₁-C₃ alkyle, et -P(O)(OC₁-C₃ alkyle)₂.

3. Composé selon la revendication 1 ou 2,
R¹ étant hydrogène ;
R² étant choisi dans le groupe constitué par -C(O)C₁-C₃ alkyle, -C (O) OC₁-C₃ alkyle, et -P (O) (OC₁-C₃ alkyle)₂ ;
R³ étant C₁-C₈ alkyle, C₁-C₈ alkyle étant éventuellement substitué par -OH, -NH₂, ou halogène ;
R⁴ étant hydrogène ;
R⁵ étant indépendamment, en chaque occurrence, C₁-C₃ alkyle ou -OC₁-C₃ alkyle ; et
m étant 2.

4. Composé selon l'une quelconque des revendications 1 à 3, R⁵ étant indépendamment, en chaque occurrence, choisi parmi hydrogène et halogène, plus particulièrement F, à la condition que toutes les 4 occurrences de R⁵ ne soient pas hydrogène, ou
le composé de formule (I) étant un composé de formule III : 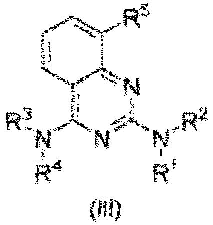 ou un sel pharmaceutiquement acceptable correspondant, R⁵ étant C₁₋₃ alkyle.

5. Composé selon l'une quelconque des revendications 1 à 4, R¹ étant hydrogène.

6. Composé selon l'une quelconque des revendications 1 à 5, R² étant -C(0)C₁-C₃ alkyle ou étant -C(O)OC₁-C₃ alkyle.

7. Composé selon l'une quelconque des revendications 1 à 6, R³ étant C₁-C₆ alkyle ou étant C₁-C₆ alkyle substitué par -OH ou ester.

8. Composé selon l'une quelconque des revendications 1 à 7, R⁴ étant hydrogène.

9. Composé selon la revendication 1, qui est 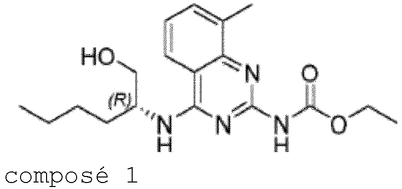 ou

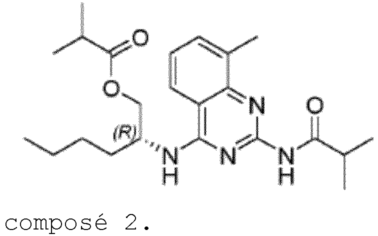

10. Composé selon l'une quelconque des revendications 1 à 9, qui est un agoniste du récepteur de type Toll (TLR), et le récepteur de type Toll étant éventuellement le récepteur 8 de type Toll (TLR8).

11. Composition pharmaceutique comprenant le composé selon l'une quelconque des revendications 1 à 10, ou un sel pharmaceutiquement acceptable correspondant, et un ou plusieurs supports ou excipients pharmaceutiquement acceptables.

12. Composé selon l'une quelconque des revendications 1 à 10, ou composition pharmaceutique selon la revendication 11, pour une utilisation dans le traitement ou la prévention d'une infection virale chez un sujet qui en a besoin.

13. Composé selon l'une quelconque des revendications 1 à 10, ou composition pharmaceutique selon la revendication 11, pour l'utilisation selon la revendication 12, l'infection virale étant une infection de type hépatite B (VHB).

14. Composé selon l'une quelconque des revendications 1 à 10, ou composition pharmaceutique selon la revendication 11, pour l'utilisation selon la revendication 12 ou 13, ledit composé ou ladite composition étant administré(e) en tant qu'adjuvant de vaccin.

15. Composé selon l'une quelconque des revendications 1 à 10, ou composition pharmaceutique selon la revendication 11, pour une utilisation dans le traitement d'un trouble immun chez un sujet qui en a besoin, ou dans le traitement d'un cancer.

16. Composé selon l'une quelconque des revendications 1 à 10, ou composition pharmaceutique selon la revendication 11, pour l'utilisation selon l'une quelconque des revendications 12 à 15, ledit composé ou ladite composition induisant une réponse à T auxiliaire 1 (Th1) chez le sujet,
la réponse à Th1 chez le sujet entraînant éventuellement la sécrétion d'IL-12p70 chez le sujet,
la réponse à Th1 entraînant éventuellement une régulation à la hausse de CD40 ou OX40L chez le sujet, et
la réponse à Th1 chez le sujet entraînant éventuellement une régulation à la hausse d'IFN_{γ} chez le sujet.

17. Composé selon l'une quelconque des revendications 1 à 10, ou composition pharmaceutique selon la revendication 11, pour l'utilisation selon l'une quelconque des revendications 12 à 16, ledit composé ou ladite composition pharmaceutique étant administré(e) oralement.
